# EUROPEAN PATENT APPLICATION

(11) **EP 2 732 824 A1**
(43) Date of publication of application: **21.05.2014**
(21) Application number: 12306428.9
(22) Date of filing: 16.11.2012
(51) Int. Cl.: A61K 38/24, A01K 67/02

(54) **Compositions and methods for increasing reproduction performance in non human mammals using alpha-beta-linked follicle stimulating hormone**

(71) Applicant: Ceva Sante Animale, 33500 Libourne Cedex (FR)
(72) Inventor: Souza, Alexandre, 33360 CARIGNAN DE BORDEAUX (FR); Isaka, Naomi, 33500 LIBOURNE (FR); Carrie, Aude, 33500 LES BILLAUX (FR); Thibaud, Dominique, 33200 BORDEAUX (FR)
(74) Representative: Becker, Philippe

(57) **Abstract**

The present invention relates to methods and compositions for increasing reproduction performance in non-human mammals using a biologically active recombinant Follicle Stimulating Hormone (rFSH). The invention also relates to methods for increasing ovulation or embryo production or pregnancies in non-human mammal using rFSH. The invention may be used in particular in ungulates such as bovine or equine.

SEQUENCE LISTING

<110>  CEVA SANTE ANIMALE

<120>  COMPOSITIONS AND METHODS FOR INCREASING REPRODUCTION PERFORMANCE
       IN NON HUMAN MAMMALS USING A BIOLOGICALLY ACTIVE RECOMBINANT
       FOLLICLE STIMULATING HORMONE (rFSH)

<130>  B1406EP

<160>  4

<170>  PatentIn version 3.3

<210>  1
<211>  109
<212>  PRT
<213>  Bovine FSH alpha subunit

<400>  1

Cys Glu Leu Thr Asn Ile Thr Ile Thr Val Glu Lys Glu Glu Cys Gly
1               5                   10                  15

Phe Cys Ile Ser Ile Asn Thr Thr Trp Cys Ala Gly Tyr Cys Tyr Thr
            20                  25                  30

Arg Asp Leu Val Tyr Arg Asp Pro Ala Arg Pro Asn Ile Gln Lys Thr
        35                  40                  45

Cys Thr Phe Lys Glu Leu Val Tyr Glu Thr Val Lys Val Pro Gly Cys
    50                  55                  60

Ala His His Ala Asp Ser Leu Tyr Thr Tyr Pro Val Ala Thr Glu Cys
65                  70                  75                  80

His Cys Ser Lys Cys Asp Ser Asp Ser Thr Asp Cys Thr Val Arg Gly
                85                  90                  95

Leu Gly Pro Ser Tyr Cys Ser Phe Arg Glu Ile Lys Glu
            100                 105

<210>  2
<211>  96
<212>  PRT
<213>  Bovine FSH beta subunit

<400>  2

Phe Pro Asp Gly Glu Phe Thr Met Gln Gly Cys Pro Glu Cys Lys Leu
1               5                   10                  15

Lys Glu Asn Lys Tyr Phe Ser Lys Pro Asp Ala Pro Ile Tyr Gln Cys
            20                  25                  30

Met Gly Cys Cys Phe Ser Arg Ala Tyr Pro Thr Pro Ala Arg Ser Lys

35                  40                  45

Lys Thr Met Leu Val Pro Lys Asn Ile Thr Ser Glu Ala Thr Cys Cys
    50                  55                  60

Val Ala Lys Ala Phe Thr Lys Ala Thr Val Met Gly Asn Val Arg Val
65                  70                  75                  80

Glu Asn His Thr Glu Cys His Cys Ser Thr Cys Tyr Tyr His Lys Ser
                85                  90                  95

<210>  3
<211>  253
<212>  PRT
<213>  Bovine rFSH analog

<400>  3

Met Lys Ser Val Gln Phe Cys Phe Leu Phe Cys Cys Trp Arg Ala Ile
1               5                   10                  15

Cys Cys Arg Ser Cys Glu Leu Thr Asn Ile Thr Ile Thr Val Glu Lys
            20                  25                  30

Glu Glu Cys Gly Phe Cys Ile Ser Ile Asn Thr Thr Trp Cys Ala Gly
        35                  40                  45

Tyr Cys Tyr Thr Arg Asp Leu Val Tyr Arg Asp Pro Ala Arg Pro Asn
    50                  55                  60

Ile Gln Lys Thr Cys Thr Phe Lys Glu Leu Val Tyr Glu Thr Val Lys
65                  70                  75                  80

Val Pro Gly Cys Ala His His Ala Asp Ser Leu Tyr Thr Tyr Pro Val
                85                  90                  95

Ala Thr Glu Cys His Cys Ser Lys Cys Asp Ser Asp Ser Thr Asp Cys
            100                 105                 110

Thr Val Arg Gly Leu Gly Pro Ser Tyr Cys Ser Phe Arg Glu Ile Lys
        115                 120                 125

Glu Ser Ser Ser Ser Lys Ala Pro Pro Pro Ser Leu Pro Ser Pro Ser
    130                 135                 140

Arg Leu Pro Gly Pro Ser Asp Thr Pro Ile Leu Pro Gln Phe Pro Asp
145                 150                 155                 160

Gly Glu Phe Thr Met Gln Gly Cys Pro Glu Cys Lys Leu Lys Glu Asn
                165                 170                 175

Lys Tyr Phe Ser Lys Pro Asp Ala Pro Ile Tyr Gln Cys Met Gly Cys
            180                 185                 190

Cys Phe Ser Arg Ala Tyr Pro Thr Pro Ala Arg Ser Lys Lys Thr Met
        195                 200                 205

Leu Val Pro Lys Asn Ile Thr Ser Glu Ala Thr Cys Cys Val Ala Lys
    210                 215                 220

Ala Phe Thr Lys Ala Thr Val Met Gly Asn Val Arg Val Glu Asn His
225                 230                 235                 240

Thr Glu Cys His Cys Ser Thr Cys Tyr Tyr His Lys Ser
                245                 250

<210>  4
<211>  762
<212>  DNA
<213>  Bovine rFSH analog

<400>  4
atgaagtctg tccagttctg tttccttttc tgttgctgga gagcaatctg ctgcagaagc      60

tgcgagctga ccaacatcac catcacggtg gagaaagagg aatgtggctt ctgcataagc     120

atcaacacca cgtggtgtgc aggctactgc tacacccggg acttggtgta cagggaccca     180

gcaaggccca atatccagaa aacgtgtacc ttcaaggagc tggtctacga gacggtgaaa     240

gtgcctggct gtgctcacca tgcagactcc ctgtacacgt acccagtagc cactgaatgt     300

cactgcagca agtgcgacag cgacagcact gactgcaccg tgagaggcct ggggcccagc     360

tactgctcct tcagggaaat caaagaatcc tcttcctcaa aggcccctcc cccgagcctt     420

ccaagtccat cccgactccc ggggccctcg gacaccccga tcctcccaca atttcctgat     480

ggagagttta caatgcaggg ctgtcctgaa tgcaagctaa aagaaaacaa atacttctcc     540

aagccagatg ctccaatcta tcagtgcatg gggtgctgct tctccagggc ataccccact     600

ccagcgaggt ctaagaagac aatgttggtc cccaagaaca tcacctcgga agctacatgc     660

tgtgtggcca aagcatttac caaggccaca gtgatgggaa atgtcagagt ggagaaccac     720

accgagtgcc actgcagcac ttgttattat cacaaatcct aa                        762

## Description

The present invention relates to methods and compositions for increasing reproduction performance in non-human mammals using a biologically active recombinant Follicle Stimulating Hormone (rFSH). The invention also relates to methods for increasing ovulation, embryo production, or pregnancies in non-human mammal using rFSH. The invention may be used in particular in ungulates such as bovine or equine, in artificial insemination or *in vitro* fertilization programs.

### BACKGROUND OF THE INVENTION

The ability to increase reproductive performance in non-human mammals such as cattle, horses or other ungulates can have a significant benefit to owners. This can be achieved through increasing fertility and/or fecundity in such non-human mammals. Currently, several methods or protocols are proposed to increase reproductive performance, based on the use of estradiol and other hormones such as GnRH (Gonadotropin Releasing Hormone), LH (Luteinizing Hormone), FSH (Follicle stimulating Hormone), or progestagens (such as progesterone) to mimic natural or close to natural levels of hormones occurring at the target specie. Some of these protocols include a step to synchronize ovulation in females to facilitate stimulation, growth and ovulation of follicles in a synchronized fashion allowing the fixed-time artificial insemination, avoiding the necessity of estrus detection. These so called synchronization protocols are widely used in commercial dairy and beef herds worldwide.

One of the most common treatments used to synchronize the emergence of follicular waves involves the use of different types of estrogens. However, this steroid hormone cannot be used in many parts of the world and alternative methods have been developed to control follicular and luteal phases aiming for instance the improvement of superovulation process of embryo-donor cows in countries where estradiol is not available. An approach that seems to be a promising option is to initiate FSH treatments at the time of the emergence of a new follicular wave, following a GnRH-induced ovulation.. In addition, because the half-life of currently available FSH products are generally short (approximately 6h) superovulatory protocols involve twice a day FSH treatments throughout 4 to 5 days, which are fairly time consuming, stressful and subject to error. Furthermore, these frequent managements during currently developed superovulation schemes make it barely impossible its application for example in beef cattle kept in pastures. Therefore, the standard superovulatory approach is based on multiple injections of FSH at the time of emergence of follicular waves that normally happens 8 to 12 days after naturally occurring estruses. This method is known as "estrus-based", and while efficient in terms of embryo production, is conditioned by the stage of the estrus cycle and requires estrus detection.. Later studies have found that fertilization rates are significantly lower in "estrus-based" programs as compared to modem synchronization protocols that allow a more ideal interval from insemination to ovulation.

Studies have been conducted in order to determine whether lengthening the FSH stimulation protocol could increase the ovulatory response. The authors appear to conclude that extending the FSH injection period from 4 consecutive days to 7 consecutive days could sustain follicle growth and result in more follicles acquiring the capacity to ovulate. More studies are ongoing to confirm this hypothesis in different cattle breeds.

In another reported protocol, cows were treated with FSH during 3 days instead of 4, and the last 2 injections, on day 4, were replaced by 2 eCG (equine Chorionic Gonadotropin) injections (Barros et al., 2008). This treatment appeared to increase the number of embryos, although the time of treatment is still long. In addition, eCG is a fairly long molecule and have been described to induce antibody formation when repeatedly used, and perhaps limiting the wide/frequent use of these types of protocols.

In the attempt to reduce the number of FSH injections, an alternative protocol has been developed wherein FSH is embedded in a slow release polymer (hyaluronan). Using this slow release formulation, a single injection was made after "estrus-based" superovulation schedules. However, the authors conclude that the hyaluronan is either too viscous and that the single intramuscular injection resulted in a lower superovulatory response (Bo et al., 2010).

EP2134165 proposes an alternative method for increasing reproduction using a single-chain FSH wherein both subunits are linked covalently by a peptide linker, which is administered in a single dose 7 to 13 days after estrus. According to this method, the animals are prepared for the FSH injection by identifying the reference estrus (or heat) date (day of last heat) and then the FSH is administered in a single injection between days 7 to 8 of the animal's cycle. The results appear satisfactory, but an individual observation and monitoring of the animals is required to determine the most effective treatment regimen.

### SUMMARY OF THE INVENTION

The present invention relates to methods and compositions for increasing reproductive performance in non-human mammals using a biologically active recombinant Follicle Stimulating Hormone (rFSH). The present invention also provides improved methods for increasing reproduction, particularly fertility and fecundity, in non-human mammals.

More particularly, the invention provides improved rFSH-based compositions and treatment methods which provide improved reproductive performance in non-human mammals by minimizing the number of administrations and the dosage.

A particular object of the invention relates to a recombinant rFSH analog, or to a composition comprising a rFSH analog, for use for increasing reproductive performance in non-human mammals undergoing a follicular synchronization protocol and/or treatment. More preferably, the rFSH analog is administered in one single administration, preferably at a dose range comprised between about 0.01 µg and about 5 mg.

Another object of the invention resides in a rFSH analog (or a composition comprising a rFSH analog) for use to increase reproductive performance in one or several non-human mammals, wherein said rFSH analog is administered to each of said one or several non-human mammals after follicle synchronization, in one single administration, preferably at a dose range comprised between 10µg and 1 mg.

Another object of the invention resides in a method for increasing reproductive performance in one or several non-human mammals, comprising administering to each of said one or several non-human mammals, after follicle synchronization, one single administration of a rFSH analog, preferably at a dose range comprised between 10µg and 1mg.

A further object of the invention is a method for inducing superovulation in one or several non-human mammals, comprising administering to each of said one or several non-human mammals a rFSH analog, wherein said rFSH analog is administered to said non-human mammals after follicle synchronization protocol and/or treatment, in one single administration, preferably at a dose range comprised between 10µg and 1mg.

Another object of the invention is a method for increasing ovulation rate in one or several non-human mammals, comprising administering to each of said one or several non-human mammals a rFSH analog, wherein said rFSH analog is administered to said non-human mammals in one single administration, preferably at a dose range comprised between 10µg and 1mg. Administration is preferably performed after follicle synchronization.

Another object of the invention is an improved method for producing or recovering oocytes in one or several non-human mammals, comprising administering to each of said one or several non-human mammals a rFSH analog, wherein said rFSH analog is administered to said non-human mammals in one single administration, preferably at a dose range comprised between 10µg and 1mg.

Administration is preferably performed after follicle synchronization. Such a method is particularly advantageous for improving the performance of *in vitro* fertilization (IVF) protocols or procedures.

In this respect, a further object of the invention relates to an *in vitro* fertilization (IVF) method comprising a step of obtaining oocytes from a non-human mammal donor and a step of fertilizing *in vitro* said oocytes, wherein the non-human mammal donor is treated by one single administration of a rFSH analog, preferably at a dose range comprised between 10µg and 1mg, to increase oocyte production. Administration is preferably performed after follicle synchronization of the non-human mammal donor.

Another object of the invention is a method for improving the quality and quantity of *Corpora Lutea* in a non-human mammal, comprising administering to said non-human mammal a rFSH analog, wherein said rFSH analog is administered to said non-human mammal in one single administration, preferably at a dose range comprised between 10µg and 1mg.

In a particular embodiment, the invention relates to a method comprising:
(a) providing a non-human mammal (e.g., an ungulate), or a group of non-human mammals (e.g., of ungulates), which has been treated to synchronize follicles; or treating a non-human mammal (e.g., an ungulate), or a group of non-human mammals (e.g. of ungulates), to synchronize follicles;
(b) administering to said non-human mammal or group of mammals one single dose of a rFSH analog at a dose range comprised between about 0.01 µg and about 5 mg, preferably by injection, more preferably by intramuscular injection; and
(c) inseminating the non-human mammal or group of non-human mammals, preferably by artificial insemination, preferably near the time of ovulation and, more preferably, 2 to 7 days, even more preferably 4 to 6 days after administration step (b).

This method avoids the need to detect or monitor estrus, and is more effective especially for groups of non-human mammals.

In another particular embodiment, the invention relates to a method comprising:
(a) providing an ungulate or a group of ungulates which has been treated to synchronize follicles; or treating an ungulate, or a group of ungulates, to synchronize follicles;
(b) administering to said ungulate or group of ungulates one single dose of a rFSH analog at a dose range comprised between about 10µg and about 1mg, preferably by injection, more preferably by intramuscular injection;
(c) recovering oocytes from the ungulate, preferably by follicular aspiration;
(d) optionally, in vitro fertilizing the recovered oocytes; and
(e) optionally implanting the fertilized oocytes in a recipient animal, preferably in a recipient animal previously treated by administration of one single dose of a rFSH analog at a dose range comprised between about 10µg and about 1mg.

The rFSH analog for use in the present invention is preferably a single chain rFSH. Furthermore, the rFSH analog is preferably used in essentially pure form, optionally in association with one or several pharmaceutically acceptable excipients or carriers.

The invention may be used in non-human mammals and preferably in any ungulate, such as bovine, ovine, equine, sheep, or goats. The compositions and method of the invention are particularly effective for increasing fertility and/or fecundity and, especially, superovulation, ovulation rate, oocyte production, or *Corpora Lutea* (CL) quality and quantity in bovine.

### LEGEND TO THE FIGURES

Figure 1: Protocol used to increase reproductive performance with a rFSH analog.
Figure 2: Nucleotide and amino acid sequence of a bovine rFSH analog for use in the invention. The CTP linker sequence which links the beta and alpha subunits is underlined. The underlined and bold nucleotides encode the signal peptide.

### DETAILED DESCRIPTION OF THE INVENTION

The present invention provides methods to increase reproductive performance in non-human mammals, and preferably ungulates, even more preferably cattle. In particular, a recombinant single chain FSH is used to stimulate fertility and/or fecundity in non-human mammal females, as illustrated by a superovulation, an increase of embryo production, and/or an increase of pregnancy. The recombinant single chain FSH is also effective to stimulate the growth and the maturation of follicles, resulting in an improved oocyte recovery, superovulation, and ovulation rate, and in an improved quality and quantity of *Corpora Lutea* (CL).

The recombinant single chain FSH can be used in insemination protocols and/or treatments, including for embryo transplantation and in vitro fertilization. The invention may be used with any ungulate, preferably bovine.

### Definitions

Within the context of the present invention, the term "increasing reproductive performance" or "increased reproductive performance" refers to increasing the likelihood that a non-human mammal, or a plurality of non-human mammals, will be fertile and fecund.

Increasing reproductive performance includes a stimulation of the growth and/or maturation of follicles, or an improvement in the quality and quantity of *Corpora Lutea.* Increased reproductive performance also includes an increased likelihood that a non-human mammal, or a plurality of non-human mammals, which has been inseminated will become pregnant, will deliver a live offspring, or develop viable embryos. Increasing reproductive performance also includes increasing the number of viable embryos a non-human mammal or a plurality of non-human mammals produce *in utero* and/or *in vitro.* Increased reproductive performance includes increasing fertility, fecundity, superovulation, oocyte rate, ovulation rate, embryo production and/or pregnancies. An increase is preferably by approximately at least 1% as compared to non-treated non-human mammals, more preferably by at least 2%, 3%, 4%, 5%, 10% or more.

The term "fertility" or "fertile" refers, within the context of this invention, to the ability to produce fertilizable oocytes.

The term "fecund" or "fecundity" refers, within the context of this invention, to the ability to complete a pregnancy.

The term "superovulation" refers, within the context of this invention, to an increase in the number of ovulated follicles and/or in the creation of fertile ova.

The term "pregnant" refers to a non-human mammal or to a group of non-human mammals some of which being currently pregnant or that has been inseminated and may be pregnant.

As used herein, the term "estrus" refers to the period during which a non-human mammal is most likely to become pregnant. Estrus may be detected or monitored by behavioral demonstration that a non-human mammal is in heat, including showing standing heat.

"Insemination" refers to introducing semen by any method known in the art, including, but not limited to, natural and Artificial Insemination (AI) and *in vitro* fertilization (IVF).

A "group" of animals designates any group of at least 2 non-human mammals, such as a herd or flock.

An "ungulate" refers to any animal with hooves and especially the two taxonomic orders Perissodactyla and Cetartiodactyla.

The term "administration" refers to all route of administration such as oral, enteral mucosal, parenteral or percutaneous. Preferably the administration route is an injection.

The term "follicle synchronization" refers, within the context of this invention, to synchronization of the emergence of follicular waves.

### Recombinant FSH analog

Follicle Stimulating Hormone belongs to the class of fertility hormones. FSH is composed of two chains (or subunits), termed alpha and beta. Under physiological conditions, both subunits are linked by non-covalent interaction. The nucleotide and amino acid sequences of FSH from distinct species have been disclosed and are available in public databases such as bovine FSH (Kim KE et al., 1988, "nucleotide sequence of the bovine gene for follicle-stimulating hormone beta-subunit", DNA1988, 7(4): 227-233) or human FSH: gene bank number: CAA43996.1.

The present invention utilizes a FSH analog. The term "analog" designates, generally, a compound which mimics the physiological effect of a natural compound. An analogs is structurally similar to the natural compound, and may present structural differences as a result of, e.g., production methods or because the differences confer a beneficial activity to the analog.

In a preferred embodiment, a FSH analog of the invention is a FSH having both alpha and beta subunits covalently linked. Such preferred FSH analog may also be designated "single chain" FSH.

In a preferred embodiment, the invention uses a recombinant FSH in which the alpha subunit is covalently linked to the beta subunit by a peptide linker. The invention shows such recombinant single chain analog of FSH provides improved properties for increasing reproductive performance in ungulates. The peptide linker may be any peptide linker which does not affect the conformation or activity of FSH. In a preferred embodiment, the linker is a CTP linker, e.g., a linker which comprises a sequence of the carboxy terminal peptide of human chorionic gonadotropin as described in US6,242,580 and US2008/0312151. In another embodiment, the linker is a peptide comprising the (G₄S) sequence, which may be repeated several times.

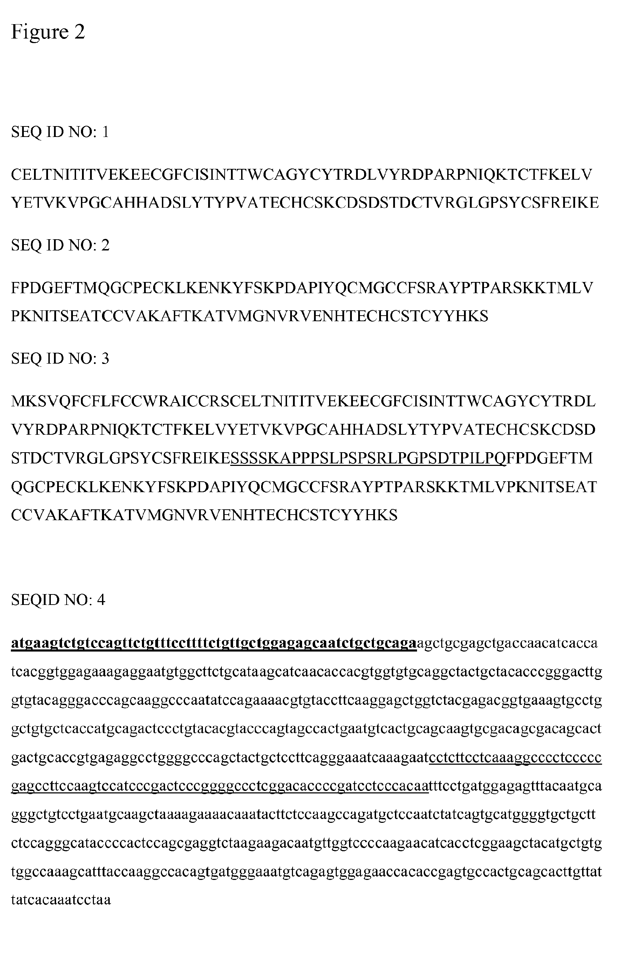

The cDNA and amino acid sequence of a recombinant bovine FSH analog for use in the present invention is provided in Figure 2 (SEQ ID NO: 3 and SEQ ID NO: 4). The first part (amino acids 1-129) corresponds to the sequence of bovine FSH beta subunit (SEQ ID NO 2), the central part corresponds to the carboxy terminal peptide linker (amino acids 130-157), and the third part (amino acids 158-253) corresponds to the sequence of bovine FSH alpha subunit (SEQ ID NO 1, see also, EP2134165).

One embodiment of the invention uses a single chain recombinant bovine FSH having the amino acid sequence of SEQ ID NO 3, or an amino acid sequence having 90% or greater, preferably 95% or greater, homology to the amino acid sequence of SEQ ID NO 3.

When the method is for treating a bovine, it is preferred to use a bovine FSH analog as disclosed above, i.e., a recombinant FSH wherein the alpha and beta domains derive from bovine FSH and are linked by a peptide linker.

When the method is for treating another ungulate, it is preferred to use a FSH analog derived from said ungulate.

The protein is preferably essentially pure, i.e., with a purity level of at least 95%, more preferably at least 97, 98 or 99%.

In a preferred embodiment, the FSH is administered in a composition comprising a suitable pharmaceutical formulation. The pharmaceutical formulation may comprise one or several excipients or carriers

In one preferred embodiment, the bovine FSH has an activity above 10 000IU/mg; more preferably above 13 000IU/mg, such as of about 16 000IU/mg. According to the conversion factor published by the ASRM practice committee (Fertility and Sterility, vol90, supl3, November 2008- American Society for Reproductive Medecine), 10 000IU of bovine FSH correspond to 600µg.

### Treatment

As previously indicated, the invention relates to novel methods for increasing reproductive performance in non-human mammals using a recombinant FSH analog. The invention may be used in insemination programs particularly ungulates artificial insemination, e.g., to improve ovulation rate and/or superovulation and/or the quality and quantity of *Corpora Lutea* in the treated animals; as well as *in vitro* fertilization programs, e.g., to improve oocyte recovery from donor animals and/or pregnancy rate in recipients animals.

More particularly, an object of the invention relates to a recombinant FSH analog for use to increase reproductive performance in non-human mammals, wherein said recombinant FSH comprises an alpha subunit and a beta subunit that are covalently linked by a peptide linker, and wherein said recombinant FSH is administered to said non-human mammals after follicle synchronization in one single administration, preferably at a dose comprised between 10µg and 1mg.

Another object of the invention resides in a method for increasing reproductive performance or embryo production in non-human mammals, comprising administering to said non-human mammals, after follicle synchronization, one single administration of a recombinant FSH analog at a dose comprised between 10µg and 1mg, said recombinant FSH analog comprising an alpha subunit and a beta subunit that are covalently linked by a peptide linker.

Another object of the invention resides in a method for improving oocyte recovery in non-human mammals, comprising administering to said non-human mammals, after follicle synchronization, one single administration of a recombinant FSH analog at a dose comprised between 10µg and 1mg, said recombinant FSH analog comprising an alpha subunit and a beta subunit that are covalently linked by a peptide linker.

Another object of the invention resides in a method for increasing pregnancy rate in non-human mammals, comprising administering to said non-human mammal, after follicle synchronization, one single administration of a recombinant FSH analog at a dose comprised between 10µg and 1mg, said recombinant FSH analog comprising an alpha subunit and a beta subunit that are covalently linked by a peptide linker.

Another object of the invention resides in a method for increasing ovulation rate in non-human mammals, comprising administering to non-human mammals, after follicle synchronization, one single administration of a recombinant FSH analog at a dose comprised between 10µg and 1mg, said recombinant FSH analog comprising an alpha subunit and a beta subunit that are covalently linked by a peptide linker.

A further object of the invention is a method for inducing superovulation in non-human mammals, comprising administering to a non-human mammal a recombinant FSH analog, wherein said recombinant FSH analog comprises an alpha subunit and a beta subunit that are covalently linked by a peptide linker, and wherein said recombinant FSH analog is administered to said mammal after follicle synchronization in one single administration, preferably at a dose comprised between 10µg and 1mg.

In a preferred embodiment, the method comprises:
(a) treating a non-human mammal (such as an ungulate) or a group of non-human mammals (such as a group of ungulates) to synchronize follicles, or providing a non-human mammal (such as an ungulate) or a group of non-human mammals (such as a group of ungulates) which has been treated to synchronize follicles;
(b) administering to the treated non-human mammal(s) between 10µg and 1mg of a recombinant FSH analog in one single dose; and
(c) optionally, inseminating the non-human mammal(s) and/or collecting oocytes preferably near the time of ovulation and, more preferably, 2 to 7 days, even more preferably 4 to 6 days after administration step (b).

In a first treatment step, emergence of a new follicular wave is synchronized. The invention shows that, in combination with a recombinant FSH of the invention, such a treatment allows improved embryo production, even at lower dose of hormone and with fewer injections.

Follicular growth is not continuous in non-human mammals such as bovine, but occurs in waves (2 to 4 waves per cycle). Each wave begins approximately when the dominant follicle in the previous wave gains maximal size, at which time numerous small follicles begin a period of rapid growth. From this group of follicles, one follicle is allowed to grow to a much larger size than the others. This large follicle is called the dominant follicle, because it has the ability to regulate and restrict the growth of the smaller follicles, called subordinate follicles. A few days after reaching maximum size, the dominant follicle begins to regress and die. As the dominant follicle degenerates, its ability to restrict the other follicles is reduced; therefore, a new follicular wave is initiated. A consequence of this dynamic process is that follicles of all sizes, including at least one large follicle, exist on each day of the estrous cycle.

Follicle wave synchronization gives the opportunity to treat all non-human mammals in a limited period of time and, therefore, to capture the economic benefits of the insemination. Upon synchronization of the estrous cycle, a high percentage of treated females show a fertile, closely synchronized estrus and ovulation.

The synchronization of ovulation or timed-AI protocols refers to methods and/or protocols that artificially stimulate follicle growth and timed ovulation, so that ovulation is initiated at a predetermined time with no need to monitor estrus behavior. A review of common methods and protocol applied in bovine are described in the article of Bo et al in the 28th Annual meeting AETE- Saint Malo, France, 7-8th September 2012 (Recent advances in the control of follicular development and superovulation protocols in cattle).

A new follicle wave emergence can be performed by hormonal or physical treatment. Hormonal treatment comprises the administration of suitable hormones such as prostaglandin(s), progestagen(s), or GnRH. Physical treatment includes follicle ablation.

In a preferred embodiment, the follicle synchronization is obtained by hormonal treatment of the non-human mammal(s), preferably with progestagens, progestagen-prostaglandin combinations, prostaglandins alone, progestagen-estrogen combinations, and gonadotropin-prostaglandin combinations with or without progestagens.

In a preferred embodiment, follicle synchronization is obtained by one of the following treatments:
- PGF2alpha or its analogs;
- GnRH + PGF2alpha
- Progestagen (as progesterone...), optionally in combination with estrogen or PGF2alpha or GnRH.

Specific dosages and/or protocols are disclosed in the art such as, e.g., in Thatcher et al., 2001 (American Association of Bovine Practitioner, AABP, Vancouver, 95-105); Diskin et al., 2001 (occasional publication n° 26, p175, British society of Animal Science); or Pursley et al., 1995 (Theriogenology 44 p915). Also, progestagens may be administered using specific devices such as implants (e.g., PRID, of Ceva Santé Animale).

In another embodiment, follicle synchronization is obtained by follicular ablation. Ablation of follicle designates the elimination, removal or destruction of at least one follicle. Follicular ablation refers to physical methods of follicular ablation, such as cautery or ultrasound-guided transvaginal follicle aspiration at random stages of the estrous cycle (Bergfelt, 1997). Ablation can be directed to all follicles or only to the one or two largest follicles (Baracaldo et al., 2000), to ensure at least the dominant follicle is suppressed.

In a preferred embodiment, the invention relates to a method comprising:
(a) treating a non-human mammal (such as an ungulate) or a group of non-human mammals (such as ungulates) with a progestagen, a progestagen-prostaglandin combination, a prostaglandin, a progestagen-estrogen combination, or a gonadotropin-prostaglandin combination with or without progestagens, to synchronize follicles; or providing a non-human mammal (such as an ungulate) or a group of non-human mammals (such as ungulates) which has been treated with a progestagen, a progestagen-prostaglandin combination, a prostaglandin, a progestagen-estrogen combination, or a gonadotropin-prostaglandin combination with or without progestagens, to synchronize follicles;
(b) administering to the treated non-human mammals (such as ungulates) between 10µg and 1mg of a recombinant FSH analog in one single dose; and
(c) optionally, inseminating a treated non-human mammal (such as ungulate) of (b) and/or collecting oocytes from a treated non-human mammal (such as ungulate) of (b), preferably near the time of ovulation and, more preferably, 2 to 7 days, even more preferably 4 to 6 days after administration step (b).

In step (b), the recombinant FSH analog can be administered using any means or techniques known per se in the art including, without limitation, systemic administration, such as intramuscular, intravenous, subcutaneous, etc. Preferred administration route is intramuscular injection.

In a preferred embodiment, the composition or method of the invention use a single dose of rFSH analog of 50 µg.

In another preferred embodiment, the composition or method of the invention use a single dose of rFSH analog of 100µg.

The results presented in the experimental section show that, on synchronized non-human mammal as ungulates, such a single administration causes production of fertile embryos and increases substantially the reproductive activity.

In a preferred embodiment, the method further comprises a step (c) of inseminating said ungulate, preferably near the time of ovulation and, more preferably, 2 to 7 days, even more preferably 4 to 6 days after rFSH analog administration.

Additional hormones, such as luteinizing hormone, chorionic gonadotropin and prostaglandin, are optionally administered as well as the rFSH. In one embodiment, prostaglandin is administered to the non-human mammal in addition to administration of the rFSH analog. The prostaglandin is optionally administered as a single dose, typically by injection, or as multiple doses administered several hours apart. In one embodiment, a first dose of prostaglandin is given to the non-human mammal after administration of the rFSH analog followed by a second dose of prostaglandin which is given to the non-human mammal approximately 6 hours to 1 day following the first prostaglandin dose.

Alternatively, the method comprises a step (c) of recovering or collecting oocytes from a treated non-human mammal of (b), preferably near the time of ovulation and, more preferably, 2 to 7 days, even more preferably 4 to 6 days after rFSH analog administration. Oocytes may be recovered by techniques known per se in the art such as, without limitation, follicular aspiration. The collected oocytes display improved characteristics for fecundity. The collected oocytes may be fertilized *in vitro,* and subsequently transferred to recipient non-human mammals, e.g., ungulates.

The invention may be used in any ungulate, such as bovine, sheep, goats, cervids, yaks, water buffaloes, bison, antelopes, gazelles, elk, reindeer, moose, bighorn sheep, giraffes, camelids, swine, equine, alpacas, and vicunas. It is particularly suited for treating female beef and dairy cattle, including heifers.

Further aspects and advantages of the invention will be disclosed in the following experimental section, which illustrates the claimed invention.

### EXAMPLES

### EXAMPLE 1 - EFFECT ON REPRODUCTION PERFORMANCE OF A SINGLE 50µg rFSH ANALOG ADMINISTRATION IN SYNCHRONIZED NON-HUMAN MAMMALS

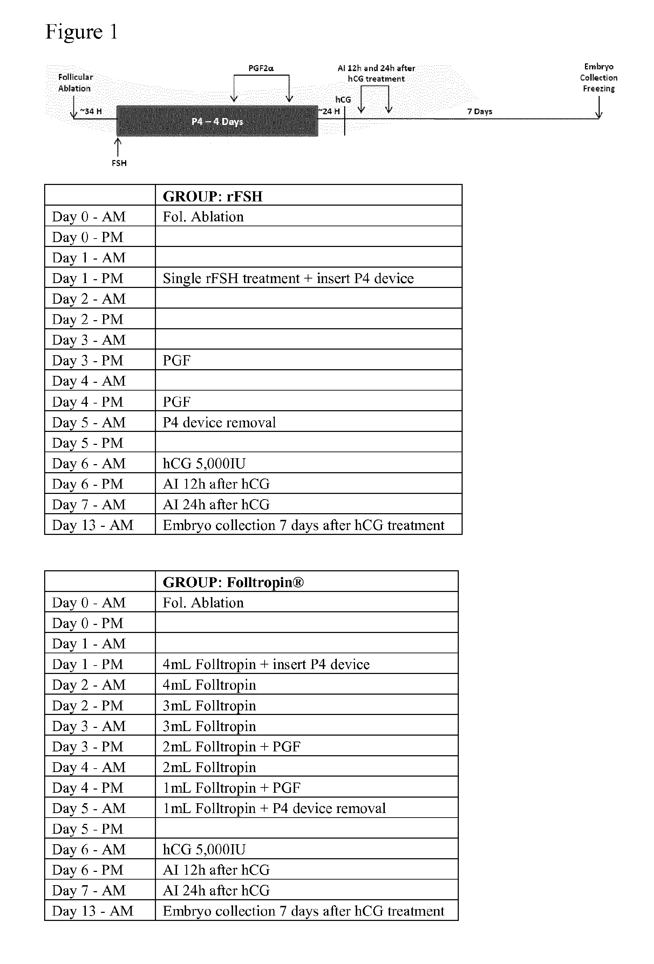

We treated 14 dairy heifers with one single intramuscular injection of bovine rFSH analog (50µg/heifer). Injection was performed 30h (in a 24 to 48 h window) after follicular ablation. The bovine rFSH analog used is as depicted in Figure 2. The treatment protocol is represented in Figure 1.

All groups received two injections of prostaglandin PGF2α at 2 days and 3 days after the first FSH injection (F0). hCG (human Chorionic Gonadotropin) injection was performed at 5 days after the F0 and Artificial Insemination (AI) was performed 12h and 24h after the hCG treatment. Embryo collection by non-surgical procedure occurred 7 days after AI.

Folltropin®-V (Bioniche Animal Health Product, Canada) was used a reference treatment. Folltropin®-V is a purified lyophilized follitropin extract obtained from porcine pituitary glands. Folltropin®-V (50 mg) was administered intramuscularly, twice daily, in decreasing doses during four days (cumulated FSH at the end of treatment is 400mg/heifer). 14 dairy heifers were treated with Folltropin®-V.

Different parameters were measured:
- Superovulation: superovulation was considered successful if > 2CL (Corpora Lutea) on the day of the flush;
- Ovulation rate: based on overall group LSM (Least Squares Means) of ovulatory response per cow;
- Embryo quality: based on overall group LSM (Least Squares Means) of number of viable embryos per cow;

The results are presented in the following table.

| | rFSH/single injection 50µg |
|---|---|
| Heifers enrolled (n) | 14 |
| Heifers superovulated (n)* | 13 |
| Heifers superovulated (%) | 92.8 |
| Ovulation rate | 81% |
| Average number of CL | 14.2 |
| Number of viable embryos (VE) | 7.9 |
| VE vs Folltropin®-V VE | 134% |

The results show that the treatment of the invention can clearly induce superovulation response in cattle. As a comparison, in the heifers treated with the reference protocol (8 injections of a natural FSH such as Folltropin®-V, 400mg), the treatment of the invention (1 single injection of a rFSH analog, 50µg) resulted in a higher ovulation rate (81% vs 76%) and a higher number of viable embryos (7.9 vs 5.9). Accordingly, even if one heifer did not superovulate with the treatment of the invention (while all did with the reference treatment), the quality of the embryos seem better with the invention. Accordingly, the combination of a follicle synchronization treatment with a single rFSH analog injection allows consistent production of superovulated embryos..

### EXAMPLE 2 - EFFECT ON REPRODUCTION PERFORMANCE OF A SINGLE 100µg rFSH ANALOG ADMINISTRATION IN SYNCHRONIZED NON-HUMAN MAMMALS

We treated 14 dairy heifers with one single intramuscular injection of bovine rFSH analog (100µg/heifer). Injection was performed 30h (in a 24 to 48 h window) after follicular ablation. The bovine rFSH analog used is as depicted in Figure 2. The treatment protocol is represented in Figure 1.

All groups received two injections of prostaglandin PGF2α at 2 days and 3 days after the first FSH injection (F0). hCG (human Chorionic Gonadotropin) injection was performed at 5 days after the F0 and Artificial Insemination (AI) was performed 12h and 24h after the hCG treatment. Embryo collection by non-surgical procedure occurred 7 days after AI.

Folltropin®-V (Bioniche Animal Health Product, Canada) was used a reference treatment. Folltropin®-V is a purified lyophilized follitropin extract obtained from porcine pituitary glands. Folltropin®-V (50 mg) was administered intramuscularly, twice daily, in decreasing doses during four days (cumulated FSH at the end of treatment is 400mg/heifer). 14 dairy heifers were treated with Folltropin®-V.

Different parameters were measured:
- Superovulation: superovulation was considered successful if > 2CL (Corpora Lutea) on the day of the flush;
- Ovulation rate: based on overall group LSM (Least Squares Means) of ovulatory response per cow;
- Embryo quality: based on overall group LSM (Least Squares Means) of number of viable embryos per cow;

The results are presented in the following table.

| | rFSH/single injection 100µg |
|---|---|
| Heifers enrolled (n) | 14 |
| Heifers superovulated (n)* | 12 |
| Heifers superovulated (%) | 85.7 |
| Ovulation rate | 88% |
| Average number of CL | 14.7 |
| Number of viable embryos (VE) | 3.8 |

The results show that the treatment of the invention can clearly induce superovulation response in cattle. As a comparison, in the heifers treated with the reference protocol (8 injections of a natural FSH such as Folltropin®-V, 400mg), the treatment of the invention (1 single injection of a rFSH analog, 100µg) resulted in a higher ovulation rate (88% vs 76%). Accordingly, even if two heifers did not superovulate with the treatment of the invention (while all did with the reference treatment), the quality of the embryos seem equivalent between the two products. The treatment achieved more than 70% of superovulatory response and also met the criteria in terms of embryo quality. Accordingly, the combination of a follicle synchronization treatment with a single rFSH analog injection allows consistent production of superovulated embryos

## Claims

1. A recombinant FSH (rFSH) analog, or a composition comprising a rFSH analog, for use to increase reproductive performance in a non-human mammal, wherein said rFSH analog is administered to said non-human mammal after follicle synchronization, in one single administration.

2. The recombinant FSH analog for use of claim 1, wherein the rFSH is administered in one single administration at a dose range comprised between about 0.01 µg and about 5 mg.

3. The recombinant FSH analog for use of claim 2, wherein the rFSH is administered in one single administration at a dose range comprised between about 1µg and 2mg, preferably between about 10µg and 1mg.

4. The recombinant FSH analog for use of claim 3, wherein the rFSH is administered in one single administration at a dose of 50µg or 100µg.

5. The recombinant FSH analog for use of anyone of claims 1 to 4, wherein said rFSH is administered by systemic administration, preferably by intramuscular administration, more preferably by injection.

6. The recombinant FSH analog for use of anyone of claims 1 to 5, wherein said rFSH analog comprises an alpha subunit having a sequence of a non- human mammal FSH alpha chain, and a beta subunit having a sequence of anon-human mammal FSH beta chain of the same species as the alpha chain, and wherein said alpha and beta subunits are covalently linked by a peptide linker.

7. The recombinant FSH analog for use of claim 6, wherein the rFSH analog is an ungulate recombinant FSH analog, preferably a bovine recombinant FSH analog comprising an bovine alpha subunit and a bovine beta subunit covalently linked by a peptide linker.

8. The recombinant FSH analog for use of claim 7, wherein the alpha subunit is a polypeptide comprising a sequence that is at least 95% homolog to SEQ ID NO 1 and the beta subunit is a polypeptide comprising a sequence that is at least 95% homolog to SEQ ID NO 2.

9. The recombinant FSH analog for use of anyone of claims 6 to 8, wherein the peptide linker is a human chorionic gonadotropin carboxy terminal peptide (CTP).

10. The recombinant FSH analog for use of anyone of claims 6 to 8, wherein the peptide linker comprises the sequence G₄S.

11. The recombinant FSH analog for use of anyone of the preceding claims, wherein the rFSH comprises an amino acid sequence having at least 95% homology with SEQ ID NO 3.

12. The recombinant FSH analog for use of any of the preceding claims, further comprising inseminating said non-human mammal near the time of ovulation and, preferably, 2 to 7 days, more preferably 4 to 6 days after rFSH administration.

13. The recombinant FSH analog for use of any of claims 1 to 11, further comprising collecting oocytes from said non-human mammal, preferably near the time of ovulation and, more preferably, 2 to 7 days, even more preferably 4 to 6 days after rFSH analog administration.

14. The recombinant FSH analog for use of anyone of the preceding claims, wherein said rFSH is injected in combination with at least one prostaglandin.

15. The recombinant FSH analog for use of anyone of the preceding claims, wherein said rFSH is injected in combination with a progestagen based- treatment.

16. The recombinant FSH analog for use of anyone of the preceding claims, comprising:
(a) providing a non-human mammal, such as an ungulate, or a group of non-human mammals, such as a group of ungulates, which has been treated to synchronize follicles;
(b) administering to said non-human mammal or group of non-human mammals one single dose of a rFSH analog at a dose range comprised between about 0.01 µg and about 5 mg, preferably by injection, more preferably by intramuscular injection; and
(c) optionally inseminating the non-human mammal or group of non-human mammals, preferably by artificial insemination, or collecting oocytes from the non-human mammal or group of non-human mammals, preferably near the time of ovulation and, more preferably, 2 to 7 days, even more preferably 4 to 6 days after administration step (b).

17. The recombinant FSH analog for use of anyone of the preceding claims, comprising:
(a) treating a non-human mammal, such as an ungulate, or a group of non-human mammals, such as a group of ungulates, to synchronize follicles;
(b) administering to said non-human mammal or group of non-human mammals one single dose of a rFSH analog at a dose range comprised between about 0.01 µg and about 5 mg, preferably by injection, more preferably by intramuscular injection; and
(c) optionally inseminating the non-human mammal or group of non-human mammals, preferably by artificial insemination, or collecting oocytes from the non-human mammal or group of non-human mammals, preferably near the time of ovulation and, more preferably, 2 to 7 days, even more preferably 4 to 6 days after administration step (b).

18. The recombinant FSH analog for use of anyone of claims 16 to 17, wherein follicle synchronization is obtained by hormonal treatment of the non-human mammal, preferably with a prostaglandin, a progestagen, and/or GnRH.

19. The recombinant FSH analog for use of claim 18, wherein follicle synchronization is obtained by a combined administration of a progestagen and prostaglandin PGF2alpha.

20. The recombinant FSH analog for use of anyone of claims 16 to 17, wherein follicle synchronization is obtained by follicular ablation.

21. The recombinant FSH analog for use of anyone of the preceding claims, wherein said analog is formulated in a composition with one or several excipients or carriers.

22. The recombinant FSH analog for use of anyone of the preceding claims, wherein the non-human mammal is an ungulate, preferably selected from a bovine, ovine or equine, more preferably a bovine.

23. The recombinant FSH analog of anyone of the preceding claims, for use in a method to increase reproductive performance in a bovine or group of bovine, wherein the method comprises:
(a) administering to the bovine, or to at least two members of the group of bovine, a prostaglandin, progestagen, and/or GnRH in an amount effective to induce follicle synchronization, and
(b) administering to the follicle-synchronized bovine, in one single administration, between 10µg and 1mg of a recombinant FSH analog.

24. A recombinant FSH analog of anyone of claims 1 to 11, for use in a method for inducing superovulation in a non-human mammal.

25. A recombinant FSH analog of anyone of claims 1 to 11, for use in a method for improving oocyte recovery in a non-human mammal.

26. A recombinant FSH analog of anyone of claims 1 to 11, for use in a method for increasing pregnancy in a non-human mammal.

27. A recombinant FSH analog of anyone of claims 1 to 11, for use in a method for increasing ovulation rate in a non-human mammal.
